(19) Europäisches Patentamt / European Patent Office / Office européen des brevets

(11) **EP 3 871 610 B1**

(12) **EUROPÄISCHE PATENTSCHRIFT**

(45) Veröffentlichungstag und Bekanntmachung des Hinweises auf die Patenterteilung:
**20.07.2022   Patentblatt 2022/29**

(21) Anmeldenummer: **21159054.2**

(22) Anmeldetag: **24.02.2021**

(51) Internationale Patentklassifikation (IPC):
*A61B 8/08* (2006.01)      *A61B 6/00* (2006.01)

(52) Gemeinsame Patentklassifikation (CPC):
**A61B 6/504; A61B 6/481; A61B 6/505; A61B 6/5217; A61B 6/5235; A61B 6/545**

(54) **VERFAHREN ZUR ÜBERLAGERUNG PROJEKTIVER ABBILDUNGEN DREIDIMENSIONALER STRUKTUREN MIT EINEM ZWEIDIMENSIONALEN RÖNTGENBILD**

METHOD FOR SUPERPOSING PROJECTIVE IMAGES OF THREE-DIMENSIONAL STRUCTURES WITH A TWO-DIMENSIONAL X-RAY IMAGE

PROCÉDÉ DE SUPERPOSITION DES REPRÉSENTATIONS PROJECTIVES DES STRUCTURES TRIDIMENSIONNELLES PAR UNE IMAGE PAR RAYON X BIDIMENSIONNELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.02.2020   DE 102020001314**

(43) Veröffentlichungstag der Anmeldung:
**01.09.2021   Patentblatt 2021/35**

(73) Patentinhaber: **Ziehm Imaging GmbH 90471 Nürnberg (DE)**

(72) Erfinder:
• **KÖNIG, Thomas 90478 Nürnberg (DE)**

• **HÖRNDLER, Klaus 90482 Nürnberg (DE)**
• **KACHELRIEß, Marc 90419 Nürnberg (DE)**
• **KNAUP, Michael 91244 Reichenschwand (DE)**

(74) Vertreter: **Engstle, Verena Meissner Bolte Patentanwälte Rechtsanwälte Partnerschaft mbB Bankgasse 3 90402 Nürnberg (DE)**

(56) Entgegenhaltungen:
**EP-A1- 3 441 003      US-A1- 2011 052 026 US-A1- 2018 042 566**

EP 3 871 610 B1

## Beschreibung

Zahlreiche bekannte medizintechnische Verfahren betreffen die Navigation von Objekten in der Anatomie eines Patienten.

Bei einer solchen Navigation von Objekten, beispielsweise beim Navigieren von Kathetern innerhalb von Gefäßen, können zur Lokalisation des Objektes röntgenbildgebende Methoden zum Einsatz kommen. Die Gefäße selbst sind dabei zunächst nicht auf den in der Regel kontinuierlich aufgenommenen Röntgenbildern zu erkennen. Aus diesem Grund wird dem Patienten - oft auch wiederholt - ein Kontrastmittel, beispielsweise ein eine Jodverbindung enthaltendes Mittel oder Kohlenstoffdioxid, verabreicht, welches eine Sichtbarmachung der Gefäßstruktur in den Röntgenbildern erlaubt.

Die gemeinsame und/oder gleichzeitige Darstellung von Objekt, Gefäßstruktur und umgebender Anatomie gestaltet sich sehr schwierig, wie im Folgenden dargestellt wird.

Für eine permanente Darstellung des Gefäßbaumes - ohne eine erneute Zugabe des Kontrastmittels - kann zunächst ein Durchleuchtungsbild des Gefäßbaumes und der ihn umgebenden Anatomie ohne Kontrastmittel aufgenommen werden (Leerbild). Anschließend wird ein Kontrastmittel injiziert und es werden zu verschiedenen, aufeinanderfolgenden Zeitpunkten Röntgenbilder angefertigt (Füllungsbilder), die die Verteilung des Kontrastmittels im Gefäß zu diesen verschiedenen, aufeinanderfolgenden Zeitpunkten dokumentieren. Die wiederholte Injektion von Kontrastmittel kann unter anderem notwendig sein, da sich das Kontrastmittel, beispielsweise ein eine Jodverbindung enthaltendes Mittel oder Kohlenstoffdioxid, im Blutkreislauf schnell verdünnt, anschließend beispielsweise in die Nieren (Jodverbindung enthaltendes Mittel) bzw. die Lunge (Kohlenstoffdioxid) transportiert und von dort aus dem Patienten wieder ausgeschieden wird. Ferner kann eine wiederholte Kontrastmittelinjektion notwendig sein, um das Gefäß in einer anderen Perspektive darzustellen, die die zu untersuchende Anatomie, beispielsweise eine Stenose, besser darstellt, oder um den Operationsfortschritt zu beurteilen.

Für die exklusive Darstellung der mit Kontrastmittel gefüllten Gefäßstrukturen wird nun das Leerbild von einem Teil der zu verschiedenen, aufeinanderfolgenden Zeitpunkten aufgenommenen Füllungsbilder subtrahiert (Subtraktionsbilder), so dass nur die mit Kontrastmittel gefüllten Gefäße als zeitliche Sequenz dargestellt werden (digitale Subtraktionsangiographie; DSA). Diese Röntgenbilder können anschließend zu einem Röntgenbild, welches einen mit Kontrastmittel voll ausgefüllten Gefäßbaum darstellt (Gefäßbild), zusammengefasst werden. Dies kann beispielsweise durch ein Minimumsverfahren (im Falle von Jod) oder ein Maximumsverfahren (im Falle von Kohlenstoffdioxid) geschehen, bei dem über den zeitlichen Verlauf der Aufnahme der einzelnen Subtraktionsbilder der jeweils niedrigste bzw. höchste Grauwert für jeden Pixel aus allen Subtraktionsbildern in den jeweiligen Pixel des Gefäßbildes überführt wird. Diese Verfahren sind aus gängigem Stand der Technik bekannt und sollen hier nicht weiter erläutert werden.

Dieses Subtraktionsbild erlaubt somit die Visualisierung von Gefäßstrukturen samt darin enthaltenen Pathologien wie beispielsweise Stenosen, Aneurysmen oder Einblutungen ins umliegende Gewebe. Die Darstellung der Gefäßstrukturen an sich ermöglicht jedoch noch keine Navigation von Objekten darin zum Zwecke eines medizinischen Eingriffs. Eine weitere Darstellung kann somit mittels eines aus dem Minimumsverfahren/Maximumsverfahren generierten und invertierten Gefäßbildes erzeugt werden. Bei einem sog., aus dem Stand der Technik bekannten, Roadmap-Verfahren kann dieses invertierte Gefäßbild mit einem Live-Röntgenbild aus der Sequenz einer im weiteren Verlauf aufgenommenen DSA überlagert und die Navigation eines Objektes in der Anatomie des Patienten vereinfacht werden. Dieses Verfahren stellt also beispielsweise den zuvor im Gefäßbild extrahierten Gefäßbaum in hellen Graustufen dar, den im Live-Röntgenbild der Sequenz der DSA sichtbaren Katheter aber weiterhin dunkel, sodass dieser während der Bewegung in der Gefäßstruktur erkannt werden kann.

Wird jedoch die Aufnahmegeometrie verändert, beispielsweise durch ein Verkippen des zur Aufnahme verwendeten Röntgensystems, so passt das zweidimensionale Subtraktionsbild nicht mehr zur neuen Aufnahmegeometrie. Eine Navigation von Objekten ist in der Regel in diesem Fall nicht ohne erneute Kontrastmittelgabe möglich. Eine Kontrastmittelgabe, insbesondere eine wiederholte Kontrastmittelgabe, stellt eine Belastung für den Patienten dar und gilt es weitestgehend zu vermeiden.

Eine Möglichkeit zur Vermeidung von weiterer Kontrastmittelgabe während eines Eingriffs zur Sichtbarmachung von kontrastarmen Strukturen ist die Verwendung eines vor dem Eingriff aufgenommenen dreidimensionalen Bilddatensatzes, welcher anatomische Informationen bzgl. der Knochen- und Gefäßstrukturen des Untersuchungsbereiches bereitstellt und somit unter Kontrastmittelgabe gewonnen wurde. Um diese im dreidimensionalen Datensatz enthaltenen Strukturen in korrekter Lage auf einem zweidimensionalen Live-Röntgenbild zum Zwecke der Navigation einzublenden, muss zunächst eine synthetische, zweidimensionale Projektion, auch Vorwärtsprojektion genannt, unter einer Projektionsgeometrie berechnet werden, die der Aufnahmegeometrie entspricht. Die Ermittlung der optimalen Projektionsgeometrie stellt dabei ein schwieriges, nicht-konvexes mathematisches Optimierungsproblem dar.

Die Herausforderung bei einer solchen Vorgehensweise ist, dass die so bestimmte optimale Projektionsgeometrie auch der tatsächlichen Projektionsgeometrie entspricht.

Bei der Verwendung von kontrastmittelfreien Röntgenbildern für die Ermittlung der optimalen Projektionsgeometrie zwischen einer aus dem dreidimensionalen Bilddatensatz erzeugten Vorwärtsprojektion und den aufgenommenen zweidimensionalen Röntgenbildern weisen insbesondere Knochen einen hohen Bildkontrast in den Röntgenbildern auf. Die alleinige Verwendung von Knochen für die Ermittlung einer Projektionsgeometrie ist hierbei möglich, für eine höhere Genauigkeit der Ähnlichkeit ist jedoch eine zusätzliche Verwendung von Gefäßen bzw. Gefäßstrukturen für die Ähnlichkeit vorteilhaft.

Ein Verfahren, welches die Ermittlung einer Koordinatentransformation zwischen einem dreidimensionalen Bilddatensatz und zweidimensionalen Röntgenaufnahmen beschreibt, ist in dem Dokument DE 10 2016 203 857 A1 offenbart. Dabei werden einzelne Registriervorschriften zwischen den Knochen- und Gefäßstrukturen aus den Live-Röntgenbildern und den Vorwärtsprojektionen aus dem dreidimensionalen Bilddatensatz verwendet.

Die Druckschriften US 2020 / 005 125 8 A1 und US 2006 / 018 813 9 offenbaren ebenfalls Verfahren für die Registrierung zwischen einem dreidimensionalen und zweidimensionalen Röntgenaufnahmen Bilddatensatz.

Die US 2011/052026 A1 offenbart ein Verfahren zum Bestimmen eines Winkels eines C-Arm-Bilderfassungssystems für eine Aortenklappenimplantation. Bei dem Verfahren werden eine oder mehrere anatomische Markierungen einer Aortenwurzel in einem 3D-Bild erfasst, und es wird auf der Grundlage der erfassten einen oder mehreren anatomischen Markierungen eine Ebene definiert, die eine Richtung eines Aortenanulus in dem 3D-Bild darstellt. Es wird ein Betrachtungswinkel bestimmt, der senkrecht zu der definierten Ebene ist.

Die US 2018/0042566 A1 offenbart ein Verfahren zur Klassifizierung eines Videos in der Koronarangiographie, wobei das Verfahren umfasst: Erfassen einer zeitlichen Reihe von angiographischen Bildframes; Entfernen eines oder mehrerer Bildframes aus der zeitlichen Reihe von angiographischen Bildern; Klassifizieren jedes der verbleibenden Bildframes der zeitlichen Reihe von angiographischen Bildern durch einen maschinell erlernten Klassifikator als eine erste anatomische Struktur oder eine zweite anatomische Struktur visualisierend; Kennzeichnen der zeitlichen Reihe von angiographischen Bildframes als die erste anatomische Struktur oder die zweite anatomische Struktur visualisierend, basierend auf den Klassifikationen der verbleibenden Bilder; und Bereitstellen der Kennzeichnung der zeitlichen Reihe für einen Benutzer.

Die Aufgabe der Erfindung ist es, ein verbessertes Verfahren zur Bestimmung einer Projektionsgeometrie zwischen einem dreidimensionalen Bilddatensatz und zweidimensionalen Röntgenbildern bereitzustellen.

Die Aufgabe der Erfindung wird erfindungsgemäß durch ein Verfahren mit den im Patentanspruch 1 angegebenen Merkmalen gelöst. Vorteilhafte Ausbildungen werden durch die abhängigen Patentansprüche angegeben.

Das erfindungsgemäße Verfahren zur Verarbeitung eines dreidimensionalen Bilddatensatzes mit zweidimensionalen Röntgenbildern eines Röntgengerätes beinhaltet dabei insbesondere die in Anspruch 1 definierten Schritte.

Der dreidimensionale Bilddatensatz, welcher anatomische Strukturen eines Untersuchungsbereiches enthält, kann durch eine präoperative Computertomographie- oder Magnetresonanztomographie-Aufnahme angefertigt werden. Die anatomischen Strukturen des dreidimensionalen Bilddatensatzes beinhalten wenigstens Knochen- und Gefäßstrukturen sowie weitere Strukturen beispielsweise Implantatsstrukturen und Hautkanten. Weiterhin kann der dreidimensionale Bilddatensatz auch intra-operativ aufgenommen werden, vorzugsweise unter Verwendung eines intra-operativen Computertomographen oder eines C-Bogen-Röntgengeräts. Ferner besteht die Möglichkeit, den dreidimensionalen Bilddatensatz in eine interne Speichereinheit, beispielsweise einen internen Bilddatenspeicher, oder eine externe Speichereinheit, beispielsweise einen USB-Stick, eine externe Festplatte, oder einen Onlinespeicher, auf welchen das das Verfahren ausführende Röntgengerät Zugriff hat, zu importieren.

Die Segmentierung des dreidimensionalen Bilddatensatzes kann vor oder nach dem Bereitstellen des dreidimensionalen Bilddatensatzes erfolgen, jeweils manuell, semi-automatisch oder vollautomatisch mittels Bildverarbeitung. Die Segmentierung stellt ein Gefäßstrukturmodell der Gefäßstruktur aus dem dreidimensionalen Bilddatensatz des Untersuchungsbereiches zur Verfügung, ebenso wird mittels der Segmentierung ein dreidimensionales Knochenstrukturmodell der Knochenstruktur aus dem dreidimensionalen Bilddatensatz des Untersuchungsbereiches zur Verfügung gestellt. Solche Modelle können beispielsweise in einer voxelweisen Segmentierung der Bildbereiche bestehen, oder aber als parametrische Repräsentation der zugrundliegenden Strukturen vorliegen. Eine parametrische Repräsentation kann beispielsweise die Beschreibung eines Gefäßes durch einen Spline für die Mittelachse des Gefäßes zzgl. eines Durchmessers an jeder Stelle des Splines sein.

Das erste und zweite Röntgenbild beschreiben die aktuelle Position des zu untersuchenden Patienten, welcher vorzugsweise auf einer Patientenliege gelagert ist, und werden mittels eines Röntgengeräts, vorzugsweise eines mobilen oder stationären mehrfach motorisch verstellbaren C-Bogen-Röntgengeräts, unter übereinstimmender Aufnahmegeometrie aufgenommen, wobei der Anwender das Röntgengerät so einstellen kann, dass die Aufnahmegeometrie den gewünschten Untersuchungsbereich erfasst. Beispielsweise kann neben der Position des Röntgengeräts die vorgenommene Einstellung den Orbitalwinkel, den Angulationswinkel, den Zoom und/oder die Höhenverstellung betreffen. Es ist vorgesehen, dass beide Röntgenbilder jeweils eine voneinander unterschiedliche Konzentration eines Kontrastmittels enthalten, beispielsweise eine jodhaltige Verbindung oder Kohlenstoffdioxid, wobei die Konzentration des Kontrastmittels für eines der beiden Röntgenbilder auch Null sein kann. Ein solches Vorgehen, wie es insbesondere aus der digitalen Subtraktionsangiographie bekannt ist, ermöglicht es durch die Subtraktion des ersten und des zweiten Röntgenbildes ein Subtraktionsbild zur Verfügung zu stellen, welches nur die Bereiche der Gefäßstruktur enthält, die durch die Hinzugabe des Kontrastmittels sichtbar gemacht werden. In der Regel wird das Kontrastmittel über die Gefäßstruktur des Patienten verabreicht, so dass es primär dessen Gefäßstruktur darstellt, insbesondere die komplette Gefäßstruktur des Patienten. Bei einer geringen Konzentration des verabreichten Kontrastmittels wird insbesondere die Knochenstruktur in den aufgenommenen Röntgenbildern dargestellt. Bei einer verwendeten Konzentration von Null des verabreichten Kontrastmittels erfolgt hauptsächlich die Darstellung der Knochenstrukturen auf dem jeweiligen Röntgenbild, auf den angerfertigten Subtraktionsbildern sind dementsprechend keine Knochenstrukturen mehr vorhanden, da auf den Subtraktionsbildern nur Gefäßstrukturen dargestellt werden.

Anschließend wird eine Projektionsgeometrie ermittelt, die eine optimale Ähnlichkeit erzielt zwischen einer aus dem dreidimensionalen Bilddatensatz erstellten zweidimensionalen Vorwärtsprojektion, diese entspricht einer zweidimensionalen Projektionsebene und dem ersten und/oder zweiten Röntgenbild sowie dem Subtraktionsbild des Untersuchungsbereiches. Die Bestimmung dieser Projektionsgeometrie kann mittels einer iterativen Vorwärtsprojektion des dreidimensionalen Bilddatensatzes und einem anschließenden algorithmischen Vergleich dieser Vorwärtsprojektion mit dem ersten und/oder zweiten Röntgenbild und dem Subtraktionsbild erfolgen.

Für diesen algorithmischen Vergleich wird eine dreiteilige Zielfunktion verwendet, wobei die drei Teile der Zielfunktion vorzugsweise additiv miteinander verknüpft sind. Dabei kann eine initiale Projektionsgeometrie, beispielsweise eine vom System vorgegebene Projektionsgeometrie, für die Bestimmung der Projektionsgeometrie verwendet werden. Diese initiale Projektionsgeometrie kann von einer berechnenden Einheit des Röntgengeräts frei gewählt oder von einem Anwender eingestellt werden und stellt die Initialisierung des zu lösenden Optimierungsproblems dar.

Ein erster Teil der Zielfunktion repräsentiert die Ähnlichkeit des Knochenstrukturmodells zwischen der Vorwärtsprojektion des dreidimensionalen Bilddatensatzes und dem ersten und/oder zweiten Röntgenbild. Bei dem algorithmischen Vergleich können die Vorwärtsprojektion beziehungsweise die im dreidimensionalen Bilddatensatz kontrastmittelhaltigen und segmentierten Gefäße virtuell mit Wasser ersetzt werden, sodass nur Knochenstrukturen in dem synthetischen zweidimensionalen Bild enthalten sind. Vorzugsweise enthält das verwendete Röntgenbild bei dem Vergleich der Knochenmerkmale kein Kontrastmittel und somit hauptsächlich Knochenstrukturen.

Ein zweiter Teil der Zielfunktion beschreibt eine Ähnlichkeit zwischen der Vorwärtsprojektion aus dem dreidimensionalen Bilddatensatz und dem Subtraktionsbild, welches, wie beispielsweise aus der digitalen Subtraktionsangiographie bekannt, nur Gefäße bzw. Gefäßstrukturen beinhaltet. Vorzugsweise werden in diesem Fall nur die segmentierten, vorwärtsprojizierten Gefäße (Gefäßstrukturmodell) mit dem Subtraktionsbild verglichen. Vorzugsweise wird das erste oder zweite Röntgenbild ohne Kontrastmittel aufgenommen, so dass diese Aufnahme als Leerbild für das Subtraktionsbild verwendet werden kann.

Es besteht die Möglichkeit, bei der Verwendung unterschiedlicher Arten von Kontrastmitteln entweder die Vorwärtsprojektion oder das Subtraktionsbild einer Inversion des Kontrasts zu unterziehen. Weiterhin können im Falle schlechter Bildqualität die Gefäße in der Vorwärtsprojektion und/oder dem Subtraktionsbild algorithmisch und vollautomatisch aufbereitet werden, um eine Zielfunktion entsprechend dem erfindungsgemäßen Verfahren zu erhalten.

Ein dritter Teil der Zielfunktion beschreibt eine Ähnlichkeit zwischen der Vorwärtsprojektion aus dem dreidimensionalen Bilddatensatz und des ersten und/oder zweiten Röntgenbildes und hierbei alle Bildinformationen der aufgenommenen Röntgenbilder, welche das erste und zweite Röntgenbild und nicht nur Knochen und/oder Gefäßstrukturen betreffen. Ferner besteht die Möglichkeit, für diesen dritten Teil der Zielfunktion bei der Verwendung von Röntgenbildern, welche mittels Kontrastmittel aufgenommen wurden, die mit Kontrastmittel gefüllten Strukturen durch Wasser zu ersetzen.

Eine mögliche Form der Zielfunktion $F$ kann eine Linearkombination der drei einzelnen Teile der Zielfunktion ($F_{Knochen}$-Knochenanteile; $F_{Gefäß}$- Gefäßanteile, $F_{Gesamt}$ - alle Anteile) sein:

$$F = \alpha * F_{Knochen} + \beta * F_{Gefäß} + \gamma * F_{Gesamt}$$

Dabei stellen die realen und vorzugsweise positiven Parameter $\alpha$, $\beta$ und $\gamma$ Gewichtungsfaktoren dar, die den relativen Einfluss der einzelnen Anteile der Zielfunktion regulieren können. Die Zielfunktion $F$ kann nun durch ein mathematisches Verfahren durch Variation der Projektionsgeometrie optimiert werden, vorzugsweise durch Anwendung eines iterativen Verfahrens. Während eines jeden Iterationsschrittes kann dabei für alle Teile der Zielfunktion hinweg dieselbe Projektionsgeometrie angenommen werden. Im Anschluss können auf Basis dieser Annahme alle Werte der Teile der Zielfunktion unter Berücksichtigung der Gewichtungsfaktoren aufsummiert werden, um den Wert der Zielfunktion $F$ zu erhalten. In den weiteren Iterationen können anschließend durch das verwendete Optimierungsverfahren weitere Projektionsgeometrien ausgewertet und nach Erreichen eines Abbruch-Kriteriums die Projektionsgeometrie mit dem optimalen Wert der Zielfunktion als die optimale Projektionsgeometrie präsentiert werden.

Das verwendete Optimierungsverfahren kann dabei aus der Klasse der Gradienten-basierten Verfahren stammen (z.B. konjugierter Gradient, Broyden-Fletcher-Goldfarb-Shanno (BFGS- Verfahren), vorzugsweise jedoch der Klasse der nicht-konvexen Optimierungsverfahren angehören (z.B. Simulated Annealing, genetische Optimierung), oder einer Kombination dieser Verfahren. Die Zielfunktion und somit alle ihre Teile können eine Ähnlichkeit zwischen dem ersten und/oder zweiten Röntgenbild bzw. Subtraktionsbild und der synthetischen Vorwärtsprojektion beschreiben; in diesem Fall handelt es sich bei dem Optimierungsproblem um ein Maximierungsproblem. Gleichermaßen kann die Zielfunktion jedoch auch eine Unähnlichkeit beschreiben; in diesem Fall handelt es sich um ein Minimierungsproblem. Je nach Ausgestaltung der Zielfunktion kann entweder ein Minimierungs- oder Maximierungsverfahren herangezogen werden. Grundsätzlich ist es jedoch auch immer möglich, eine zu maximierende Ähnlichkeit, beispielsweise durch Multiplikation mit "-1", in eine zu minimierende Unähnlichkeit zu transformieren, und umgekehrt. Dies ist insbesondere daher von Bedeutung, da die meisten, in Software-Bibliotheken verfügbaren Optimierungsverfahren als Minimierungsverfahren formuliert und implementiert sind. Im Folgenden soll zur Vereinfachung daher stets von einem Optimierungsproblem die Rede sein.

Es ist möglich, diese Parameter in Abhängigkeit der Körperregion, in welcher ein Eingriff durchgeführt wird, festzulegen bzw. diese entsprechend zu verändern. Dies kann beispielsweise in einem Programm, vorzugsweise einem Organprogramm, erfolgen. So kann in Knochenstrukturbereichen, welche markante Strukturmerkmale aufweisen, beispielsweise Wirbel mit Wirbelfortsätzen, die Gewichtung von Knochenstrukturähnlichkeiten deutlich höher eingestellt werden als die Gewichtung der Gefäßstrukturähnlichkeit. Gegensätzlich dazu verhalten sich Bereiche, welche weniger markante Knochenstrukturmerkmale aufweisen, beispielsweise der Oberschenkelknochen. In diesem Fall ist eine geringere Gewichtung der Knochenstrukturähnlichkeit und/oder eine entsprechend höhere Gewichtung der Gefäßstrukturen möglich. Die Bestimmung der Gewichtungsfaktoren kann automatisch geschehen, beispielsweise mittels einer Bildanalyse der Röntgenaufnahme und einem Abgleich des Inhalts mit einer hinterlegten Datenbank, welche die Gewichtungsfaktoren bzgl. des Bildinhalts bzw. deren anatomischer Inhalte vorgibt. Ferner können die Koeffizienten auch in einem Organprogramm entsprechend tabelliert gespeichert werden. Weiterhin ist es möglich die Gewichtungsfaktoren mittels eines Benutzereingriffs individuell einzustellen. Ferner besteht die Möglichkeit, dass die Parameter auch den Wert Null annehmen können, beispielsweise durch ein Einstellen des Benutzers, durch Hinterlegung in einem Organprogramm oder durch das erfindungsgemäße Verfahren. Mit dem Nullsetzen eines Gewichtungsfaktors werden Teile der Zielfunktion für deren Bestimmung nicht mehr berücksichtigt.

Der Wert der Zielfunktion sowie deren Bestandteile können ein Maß für die Qualität der bestimmten Projektionsgeometrie darstellen. Die Zielfunktion kann somit als ein Maß der Ähnlichkeit, beispielsweise eine Deckungsgleichheit, der Vorwärtsprojektion mit dem ersten und/oder zweiten Röntgenbild oder des Subtraktionsbildes sein. Erfüllen die nach Abschluss der Optimierung erhaltenen Werte ein Schwellwertkriterium, welches mit der Zielfunktion verglichen wird, wie es entsprechend in einem Programm, beispielsweise einem Organprogramm, hinterlegt sein kann, ist die ermittelte Projektionsgeometrie als ausreichend korrekt einzustufen.

In alternativen Ausgestaltungsformen können für den Fall, dass der Wert der Zielfunkton das vorgegebene Schwellwertkriterium erfüllt, die vorwärtsprojizierten Gefäßstrukturen aus dem dreidimensionalen Bilddatensatz mit dem Live-Röntgenbild überlagert werden. Vorzugsweise können in diesem Fall die Gefäße mit oder ohne Kontur, unter Verwendung der bestimmten Projektionsgeometrie auf dem Live- Röntgenbild dargestellt werden. Die Verwendung von solchen Schwellwertkriterien kann vorteilhaft sein, da mittels dieser die Qualität der Projektionsgeometrie sichergestellt werden kann. Somit bietet die Verwendung von Schwellwertkriterien die Möglichkeit, dass bei Nicht-Erfüllung des Schwellwertkriteriums durch den Wert der Zielfunktion ein alternativer Verfahrensweg gewählt werden kann.

In alternativen Ausgestaltungsformen kann eine wiederkehrende Neuberechnung des ersten und dritten Teils der Zielfunktion (reduzierte Zielfunktion) erfolgen, welche auch ohne Kontrastmittelgabe ständig, also bei Vorliegen eines aktuellen (Live-) Röntgenbildes, zur Verfügung stehen. Wenn kein Kontrastmittel mehr im Untersuchungsbereich vorhanden ist, bleiben diese beiden Teile der Zielfunktion unverändert. Bei der Berechnung dieser beiden Teile der Zielfunktion, basierend auf neuen aufgenommenen Röntgenbildern und der zuvor mittels der erhaltenen Projektionsgeometrie erzielten synthetischen Vorwärtsprojektion, wird der neu berechnete Wert der reduzierten Zielfunktion mit dem vorherigen und gespeicherten Wert der Zielfunktion verglichen. Im Falle, dass der neu berechnete Wert der reduzierten Zielfunktion den alten Wert der Zielfunktion innerhalb einer vorgebbaren Toleranz verfehlt, beispielsweise um einen festzulegenden Faktor, welcher in einem Programm, beispielsweise dem Organprogramm, festgelegt werden kann, wird die Gefäßstruktur bzw. die Gefäßkontur nicht mehr auf dem Live-Röntgenbild dargestellt. Im Fall, dass die neu berechneten Werte der reduzierten Zielfunktion den Schwellwert wieder erfüllen, kann die Gefäßstruktur wieder eingeblendet werden. Ein Vorteil des erfindungsgemäßen Verfahrens kann somit sein, eine kontinuierliche automatisierte Risikoabschätzung dahingehend durchzuführen, dass die Vorwärtsprojektion weiterhin ausreichend der tatsächlichen Projektionsgeometrie entspricht.

In alternativen Ausgestaltungsformen ist es möglich, dass der Wert der Zielfunktion für ein gewisses Zeitintervall den Schwellwert nicht erfüllt, beispielsweise durch eine Veränderung der Aufnahmegeometrie des Röntgengerätes oder beispielsweise durch eine bekannte Veränderung der Position des Röntgengerätes bzw. Tisches und dadurch des Patienten. Das Röntgengerät kann darüber informieren und dem Anwender eine erneute Bestimmung der Projektionsgeometrie unter erneuter Kontrastmittelgabe oder alternativ das Umschalten in einen alternativen zweidimensionalen Arbeitsablauf anbieten. Die Länge dieses Zeitintervalls, in welchem ein nicht Erfüllen des Schwellwertes erlaubt ist, kann in einem Programm, beispielsweise im Organprogramm, eingestellt werden.

In alternativen Ausgestaltungsformen kann der Fall auftreten, dass die verglichenen Werte der Zielfunktion dem vorgegebenen Schwellwertkriterium nicht genügen. In diesem Fall kann das Röntgengerät eine Ausgabe auf einem Anzeigegerät, beispielsweise einem Monitor, ausgeben, welche ein Fortlaufen des Verfahrens unterbinden und dem Anwender einen alternativen, zweidimensionalen-Arbeitsablauf anbieten. Diese Vorgehensweise sorgt für eine Risikominimierung.

In alternativen Ausgestaltungsformen ist es ferner möglich, in einem Programm, beispielsweise einem Organprogramm, dass bei einer Unterschreitung (im Falle einer Maximierung) bzw. Überschreitung (im Falle einer Minimierung) von einem Schwellwert von einem Teil der Zielfunktion, von zwei Schwellwerten von zwei Teilen der Zielfunktion oder von drei Schwellwerten von drei Teilen der Zielfunktion, auf ein zweidimensionales Verfahren umgeschaltet werden kann.

In einer alternativen Ausgestaltung des Verfahrens ist vorgesehen, in jedem Live-Röntgenbild wenigstens ein Messfeld vorzusehen. Das Messfeld kann unter Benutzereingriff oder durch eine Systemvorgabe derart festgelegt sein, dass das Messfeld eine sich möglicherweise zeitlich verändernde Struktur eng eingrenzt, wobei beispielsweise eine Bewegung des Patienten eine zeitliche verändernde Struktur sein kann. Die Auswertung des Messfeldes erfolgt vorzugsweise durch Addition aller Pixelwerte im Messfeld und Überwachung des Summenwertes in einer Folge von Live-Bildern. Bei einer örtlichen Veränderung der eingegrenzten Struktur im Messfeld ändert sich der Summenwert der Pixelwerte gegenüber dem Summenwert des vorherigen Live-Röntgenbildes. Es ist vorgesehen, den Summenwert durch Schwellwertsetzung zu überwachen.

Der Schwellwert kann vorzugsweise als Bruchteil des Summenwertes des vorherigen Live-Röntgenbildes festgelegt sein. Bei einer überschwelligen Veränderung des Summenwertes gegenüber dem vorherigen Live-Röntgenbild wird eine überschwellige Verlagerung der eingegrenzten Struktur angenommen

In einer alternativen Ausgestaltung wird anstatt des einen Subtraktionsbildes eine komplette digitale Subtraktionsangiografie angefertigt, welche nicht nur einen Teil, sondern die komplette Gefäßstruktur darstellt. In einer alternativen Ausgestaltung kann ergänzend zur DSA in bekannter Weise ein Minimums- bzw. Maximumsverfahren durchgeführt werden. Bei solchen Verfahren wird über den zeitlichen Verlauf der Aufnahme der einzelnen Subtraktionsbilder der DSA der jeweils niedrigste (Minimumsverfahren) bzw. höchste (Maximumsverfahren) Grauwert für jeden Pixel aus allen Subtraktionsbildern in den jeweiligen Pixel eines Gefäßbildes überführt. Für den weiteren Verfahrensverlauf wird dann anstatt der ersten oder zweiten Aufnahme das mittels des Minimums bzw. Maximumsverfahren erzeugte Gefäßbild anstelle des Subtraktionsbildes genutzt.

In den nachfolgenden Ausführungsbeispielen wird die Erfindung genauer erläutert.

**Fig. 1** zeigt einen beispielsweisen Verfahrensablauf des erfindungsgemäßen Verfahrens.

**Fig. 2** zeigt in einer beispielhaften grafischen Darstellung die Bestimmung der optimalen Projektionsgeometrie zwischen einem aufgenommen dreidimensionalen Bilddatensatz und dem ersten und/oder zweiten Röntgenbild und dem Subtraktionsbild.

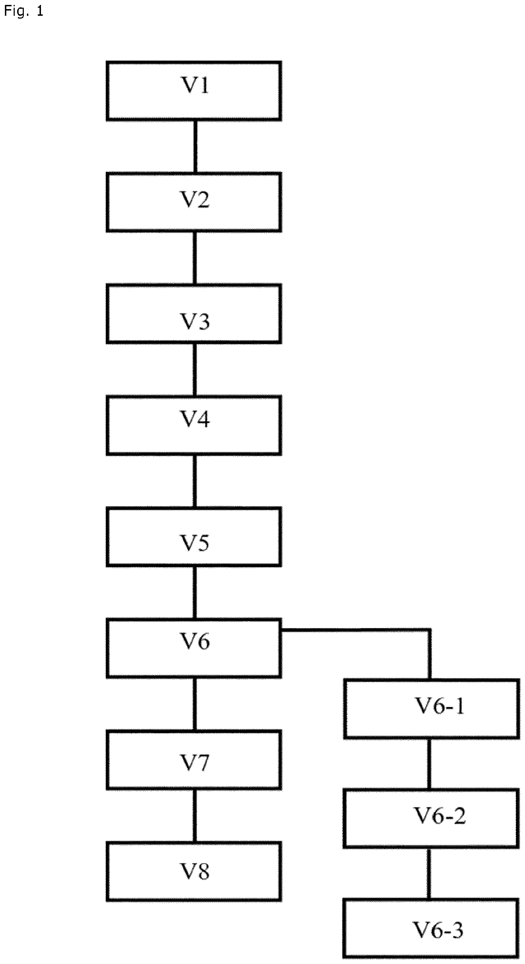

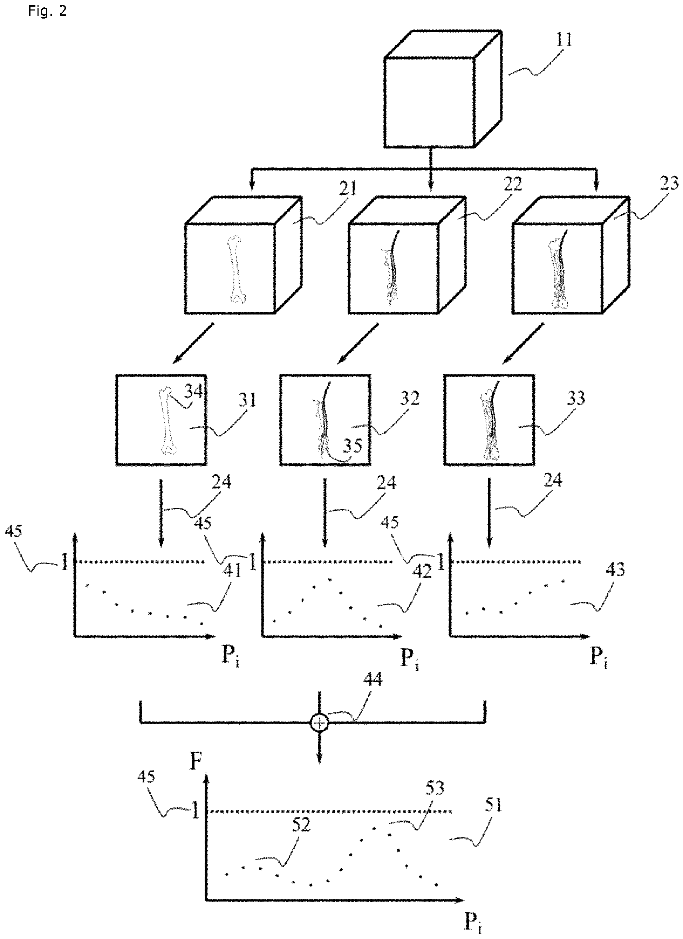

Das Verfahren wird mit Bezug zur Figur 1 und Figur 2 näher erläutert.

Während des Verfahrens kann diesem ein kontinuierliches Röntgenbild (Live-Röntgenbild) zur Verfügung gestellt werden, mit welchem zweidimensionale Gefäßstrukturen, welche aus einem zuvor aufgenommenen dreidimensionalen Bilddatensatz durch Projektion erhalten wurden, überlagert werden, um dem Anwender eine Darstellungsmöglichkeit der Gefäßstrukturen auf einem Live-Röntgenbild zur Verfügung zu stellen.

Im Verfahrensschritt V1 wird zunächst der dreidimensionale Bilddatensatz aufgenommen, beispielsweise mittels eines Computertomographen oder eines Magnetresonanztomographen. Dieser dreidimensionale Bilddatensatz umfasst dabei die Knochenstruktur und die Gefäßstruktur eines Untersuchungsbereiches. Der dreidimensionale Bilddatensatz kann präoperativ oder intraoperativ aufgenommen werden.

In einem Verfahrensschritt V2 wird anschließend der dreidimensionale Bilddatensatz in Knochenstruktur und Gefäßstruktur segmentiert, dies kann ebenfalls direkt nach der präoperativen Aufnahme oder vor dem geplanten Eingriff geschehen.

Nach der Positionierung des Patienten bezüglich des (C-Bogen-)Röntgengerätes wird in einem Verfahrensschritt V3 eine erste Röntgenaufnahme des Patienten angefertigt, dabei kann dem Patienten ein Kontrastmittel injiziert werden. Es empfiehlt sich jedoch, insbesondere die erste Röntgenaufnahme ohne ein Kontrastmittel anzufertigen, so dass die erste Röntgenaufnahme hauptsächlich nur die Knochenstruktur zeigt, samt umgebender, schwach sichtbarer Weichteilstrukturen.

In einem Verfahrensschritt V4 wird weiterhin eine zweite Röntgenaufnahme des Untersuchungsbereiches angefertigt, bei welcher dem Patienten ein Kontrastmittel injiziert wurde, so dass Teile seiner Gefäßstruktur sichtbar gemacht werden. Im Verfahrensschritt V4 kann ebenfalls eine digitale Subtraktionsangiografie durchgeführt werden. Nach einer digitalen Subtraktionsangiografie kann im Verfahrensschritt V5 ein darauffolgendes Minimums- bzw. Maximumsverfahren angewendet werden, bei welchem über den zeitlichen Verlauf der Aufnahme der digitalen Subtraktionsangiografie der jeweils niedrigste bzw. höchste Grauwert für jeden Pixel aus allen Subtraktionsbildern in den jeweiligen Pixel eines Gefäßbildes überführt wird. Für den weiteren Verfahrensverlauf wird das erzeugte Gefäßbild genutzt, welches die komplette Gefäßstruktur darstellt.

Der darauffolgende Verfahrensschritt V6 umfasst die Ermittlung der optimalen Projektionsgeometrie zwischen einer Vorwärtsprojektion des dreidimensionalen Bilddatensatzes und des ersten Röntgenbildes. Die Vorwärtsprojektion ist ein aus dem dreidimensionalen Bilddatensatz synthetisiertes zweidimensionales Röntgenbild. Es besteht ferner die Möglichkeit anstelle des ersten Röntgenbildes das zweite Röntgenbild zu verwenden.

In einem Verfahrensschritt V6-1 kann der erste Teil der Zielfunktion eine Ähnlichkeit der Knochenstruktur zwischen der Vorwärtsprojektion des dreidimensionalen Bilddatensatzes und des ersten und/oder zweiten Röntgenbildes bestimmt werden. Vorzugsweise wird in Verfahrensschritt V6-1 das Röntgenbild verwendet, bei welcher hauptsächlich die Knochenstrukturen dargestellt sind.

Für den Verfahrensschritt V6-1 kann ein algorithmischer Vergleich zwischen der Vorwärtsprojektion und des ersten Röntgenbildes verwendet werden. Bei diesem algorithmischen Vergleich kann die Ähnlichkeit aus einer Vorwärtsprojektion und des ersten Röntgenbildes maximiert werden, vorzugsweise unter Variation der Geometrie unter welcher die Vorwärtsprojektion erstellt wird, beispielsweise unter Verwendung von bis zu drei Translationen und bis zu drei Rotationen der Projektionsgeometrie. Für die Bestimmung der Ähnlichkeit der Knochenstruktur empfiehlt es sich vorzugsweise die in der Vorwärtsprojektion beziehungsweise die im dreidimensionalen Bilddatensatz kontrastmittelhaltigen und segmentierten Gefäßstrukturen virtuell mit Wasser zu ersetzen, so dass nur Knochenstrukturen in der Vorwärtsprojektion enthalten sind. Es empfiehlt sich bei der Bestimmung der Ähnlichkeit der Knochenstrukturen, dass das verwendete erste Röntgenbild vorzugsweise ebenfalls kein Kontrastmittel aufweist und somit nur Knochenstrukturen darstellt.

Im Verfahrensschritt V6-2 wird der zweite Teil der Zielfunktion bestimmt. Die Gefäßstrukturen des Gefäßbildes aus Verfahrensschritt V5 werden mit der aus dem dreidimensionalen Bilddatensatz erzeugten Vorwärtsprojektion verglichen. Die Ähnlichkeit der Gefäßstruktur zwischen der Vorwärtsprojektion des dreidimensionalen Bilddatensatzes und dem ersten und/oder zweiten Röntgenbild und des Subtraktionsbildes kann ebenfalls mittels eines algorithmischen Vergleichs unter Variation der Projektionsgeometrie berechnet werden Es empfiehlt sich im Fall der Bestimmung der Ähnlichkeit der Gefäßstruktur vorzugsweise nur die segmentierten Gefäße in der Vorwärtsprojektion mit dem Subtraktionsbild zu vergleichen.

Im Verfahrensschritt V6-3 wird eine Ähnlichkeit basierend auf allen Bildinformationen zwischen der Vorwärtsprojektion des dreidimensionalen Bilddatensatzes und der ersten und/oder zweiten Röntgenaufnahme ermittelt. Bei der Bestimmung des dritten Teils der Zielfunktion werden alle Bildinformationen der ersten und/oder zweiten Röntgenaufnahme verwendet. Für den Verfahrensschritt können beispielsweise Gewebe-zu-Luft-Übergänge, welche markante Strukturmerkmale darstellen können, verwendet werden. Die Verfahrensschritte V6-1, V6-2 und V6-3 können in ihrer Reihenfolge vertauscht werden, ohne den Verfahrensablauf zu beeinträchtigen.

Die Optimierung der Zielfunktion kann durch Anwendung eines iterativen Verfahrens durchgeführt werden. Während eines jeden Iterationsschrittes wird dabei für die Verfahrensschritte V6-1 - V6-3 (alle Teile der Zielfunktion) hinweg dieselbe Projektionsgeometrie angenommen. Im Anschluss werden auf Basis dieser Annahme alle Werte der Teile der Zielfunktion unter Berücksichtigung der Gewichtung der Teile der Zielfunktion aufsummiert, um den Wert der Zielfunktion für eine Projektionsgeometrie zu erhalten. In den weiteren Iterationen werden dann durch das verwendete Optimierungsverfahren weitere Projektionsgeometrien ausgewertet und nach Erreichen eines Abbruch-Kriteriums, beispielsweise das Erreichen der maximalen Iterationsschritte die Projektionsgeometrie mit dem optimalen Wert der Zielfunktion als die optimale Ähnlichkeit präsentiert.

Im Verfahrensschritt V7 ist es vorgesehen, im Falle einer erfolgreichen Bestimmung der Projektionsgeometrie das zweidimensionale Live-Röntgenbild mit den unter der optimalen Projektionsgeometrie vorwärtsprojizierten Gefäßstrukturen in der Ebene des Röntgenbildes zu überlagern. Beispielsweise können Gefäßstrukturen mit oder ohne Kontur oder nur die Kontur, unter Verwendung der Vorwärtsprojektion des Gefäßstrukturmodells bei der optimalen Projektionsgeometrie auf einem Anzeigegerät mit dem Live-Röntgenbild überlagert dargestellt werden.

Es ist möglich, dass sich die Lage des Untersuchungsbereiches auch während des Eingriffs ändert, beispielsweise durch eine Änderung der Aufnahmegeometrie des Röntgengerätes oder durch eine Verstellung der Tischposition. Diese Änderungen können ohne erneute Kontrastmittelgabe eine weitere Anwendung des Verfahrens ermöglichen, wobei die Möglichkeit besteht, auch ohne eine erneute Bestimmung der optimalen Projektionsgeometrie mit dem erfindungsgemäßen Verfahren fortzufahren, nur durch eine Transformation der vor der Änderung der Aufnahmegeometrie ermittelten Projektionsgeometrie. In dem Fall, dass sich die Aufnahmegeometrie ändert, können Encoder, dabei handelt es sich um Positions- und Winkelgeber des Röntgengerätes oder des Tisches ausgewertet werden und diese Informationen mit der zuvor bestimmten optimalen Projektionsgeometrie verrechnet werden.

Gemäß dem Fall, dass der neue Wert den hinterlegten Schwellwert überschreitet (im Falle eines Minimierungsproblems) bzw. unterschreitet (im Falle eines Maximierungsproblems), beispielsweise um einen festzulegenden Faktor, welcher in einem Programm, beispielsweise dem Organprogramm, festgelegt werden kann, kann die Gefäßstruktur bzw. die Gefäßkontur nicht mehr auf dem Live-Röntgenbild dargestellt werden. Im Fall, dass die neu berechneten Werte den Schwellwert wieder unterschreiten, kann die Gefäßstruktur wieder eingeblendet werden. Letzteres kann auch der Fall sein bei einem kurzfristigen Überschreiten des Schwellwertes, beispielsweise durch eine Bewegung des Operationstisches, durch eine Bewegung des Röntgengerätes oder eine dem Röntgengerät nicht angekündigte Kontrastmittelgabe. Werden die Werte innerhalb eines vordefinierten Zeitintervalls, welches in einem Programm, beispielsweise im Organprogramm eingestellt werden kann, nicht mehr unter- bzw. überschritten, kann das Röntgengerät darüber informieren und dem Anwender eine erneute Bestimmung der Projektionsgeometrie unter erneuter Kontrastmittelgabe oder alternativ das Umschalten in einen alternativen zweidimensionalen Arbeitsablauf anbieten.

Ein Umschalten auf eine alternative zweidimensionale Darstellung ist in Fig. 1 als Verfahrensschritt V8 dargestellt. In dem Fall, dass die verglichenen Werte die entsprechenden Schwellwerte nicht erfüllen, kann das Röntgengerät darüber informieren, beispielsweise durch anzeigen einer Meldung auf dem Anzeigegerät. Der Anwender kann nun das Umschalten auf den 2D-Arbeitsablauf auswählen, auch dies entspricht Verfahrensschritt V8.

Die Bestimmung der Zielfunktion und der optimalen Projektionsgeometrie wird durch die Figur 2 näher beschrieben.

In Figur 2 wird zunächst ein dreidimensionaler Bilddatensatz 11 in ein Knochenstrukturmodell 21, in ein Gefäßstrukturmodell 22 und in einen dritten Teil 23, welcher alle Bildinformationen des dreidimensionalen Bilddatensatzes 11 enthält, segmentiert. Anschließend werden jeweils aus den segmentierten Anteilen zweidimensionale synthetische Vorwärtsprojektionen ermittelt (31, 32, 33) welche mittels einer Projektionsgeometrie 24 ($P_i$) mit dem ersten und/oder zweiten Röntgenbild und dem Subtraktionsbild verglichen werden und aus dem Vergleich für jeden segmentierten Anteil (21, 22, 23) eine Ähnlichkeit bestimmt wird. Der maximale Wert für die Bestimmung der Ähnlichkeit 45 kann dabei 1 betragen, dies entspricht einer kompletten Übereinstimmung der synthetischen Vorwärtsprojektion mit dem ersten und/oder zweiten Röntgenbild und dem Subtraktionsbild. Vorzugsweise verwendet das Programm für den Vergleich zur Bestimmung der Ähnlichkeiten markante Strukturmerkmale (34, 35), beispielsweise spezielle Knochenformen 34 oder markante Gefäßverzweigungen 35.

Eine anschließende Summation 44 der Ähnlichkeiten der einzelnen segmentierten Anteile (21, 22, 23), wobei die Ähnlichkeiten der einzelnen segmentierten Anteile (21, 22, 23) jeweils mittels eines Koeffizienten gewichtet sein können, ergibt einen Wert der Zielfunktion 51. Durch weitere Iterationsschritte kann unter Variation der Projektionsgeometrie 24 für jede weitere verwendete Projektionsgeometrie 24 alle drei Teile der Zielfunktion verschiedene Ähnlichkeiten (41, 42, 43) der einzelnen segmentierten Anteile (21, 22, 23) ermittelt werden.

In den weiteren Iterationen werden dann durch das verwendete Optimierungsverfahren weitere Projektionsgeometrien ausgewertet und nach Erreichen eines Abbruch-Kriteriums, beispielsweise das Erreichen eines vorgegebenen Schwellwertes der Zielfunktion, die Projektionsgeometrie mit dem optimalen Wert der Zielfunktion als die optimale Ähnlichkeit präsentiert. Die optimale Ähnlichkeit kann das globale Maximum 53 der ermittelten Zielfunktion 51 sein, wobei neben dem globalen Optimum 53 auch lokale Extrema 52 in der Zielfunktion 51 ermittelt werden können.

## Patentansprüche

1. Computer-implementiertes Verfahren zur Verarbeitung eines dreidimensionalen Bilddatensatzes (11) mit zweidimensionalen Röntgenaufnahmen eines Röntgengerätes, beinhaltend die Schritte:

   • Bereitstellen eines dreidimensionalen Bilddatensatzes (11) wenigstens eines Untersuchungsbereiches, in welchem anatomische Strukturen vorliegen,
   • Segmentieren des dreidimensionalen Bilddatensatzes (11) für eine Zurverfügungstellung eines eine Gefäßstruktur beschreibenden dreidimensionalen Gefäßstrukturmodells (22) und eines eine Knochenstruktur beschreibenden dreidimensionalen Knochenstrukturmodells (21),
   • Aufnehmen, mittels des Röntgengeräts, eines ersten zweidimensionalen Röntgenbildes, welches wenigstens teilweise die Knochenstruktur und wenigstens teilweise die Gefäßstruktur enthält, unter einer Aufnahmegeometrie und dem Vorliegen einer ersten Kontrastmittelkonzentration,
   • Aufnehmen, mittels des Röntgengeräts, eines zweiten zweidimensionalen Röntgenbildes des Untersuchungsbereiches, welches eine zum ersten Röntgenbild unterschiedliche zweite Kontrastmittelkonzentration enthält, wobei die Aufnahme des zweiten Röntgenbildes unter der gleichen Aufnahmegeometrie wie die Aufnahme des ersten Röntgenbildes erfolgt,

• Subtraktion des ersten und des zweiten Röntgenbildes zur Erzeugung und Zurverfügungstellung eines Subtraktionsbildes, wobei das Subtraktionsbild eine Gefäßstruktur enthält,

• Ermitteln einer optimalen Projektionsgeometrie (24) unter Verwendung einer dreiteiligen Zielfunktion (51),

■ wobei ein erster Teil der Zielfunktion (51) eine Ähnlichkeit zwischen einer Vorwärtsprojektion (31; 32; 33) des Knochenstrukturmodells (21) des dreidimensionalen Bilddatensatzes (11) und der Knochenstruktur in dem ersten und/oder zweiten Röntgenbild unter Variation einer angenommenen Projektionsgeometrie ($P_i$) darstellt,

■ wobei ein zweiter Teil der Zielfunktion (51) eine Ähnlichkeit zwischen der Vorwärtsprojektion (31; 32; 33) des Gefäßstrukturmodells (22) des dreidimensionalen Bilddatensatzes (11) und der Gefäßstruktur des Subtraktionsbildes unter Variation der angenommenen Projektionsgeometrie (24) darstellt,

■ und wobei ein dritter Teil (23) der Zielfunktion (51) eine Ähnlichkeit zwischen allen Bildinformationen der Vorwärtsprojektion (31; 32; 33) des dreidimensionalen Bilddatensatzes (11) und allen Bildinformationen des ersten und/oder zweiten Röntgenbildes unter Variation der angenommenen Projektionsgeometrie (24) darstellt; wobei

• die optimale Projektionsgeometrie (24) aus einer Minimierung oder Maximierung (53) der Zielfunktion (51) hervorgeht.

2. Verfahren nach Anspruch 1, **dadurch gekennzeichnet, dass** die Zielfunktion (51) und/oder Teile der Zielfunktionen (51) mit einem vorgebbaren Schwellwertkriterium verglichen werden.

3. Verfahren nach Anspruch 2, **dadurch gekennzeichnet, dass** bei Verfehlen des Schwellwertkriteriums der Zielfunktion (51) das Verfahren abgebrochen und einem Anwender das Umschalten auf ein alternatives zweidimensionales Verfahren angeboten wird.

4. Verfahren nach einem der vorhergehenden Ansprüche, **dadurch gekennzeichnet, dass** zur Ermittlung der optimalen Projektionsgeometrie (24) die drei Teile der Zielfunktion (51) unterschiedlich gewichtet werden.

5. Verfahren nach einem der vorhergehenden Ansprüche, **dadurch gekennzeichnet, dass** anstatt einer zweiten zweidimensionalen Röntgenaufnahme des Untersuchungsbereiches eine digitale Subtraktionsangiografie erzeugt und verwendet wird.

6. Verfahren nach Anspruch 5, **dadurch gekennzeichnet, dass** die digitale Subtraktionsangiografie durch ein Minimums- oder Maximumsverfahren zusammengefasst wird.

7. Verfahren nach einem der vorhergehenden Ansprüche, **dadurch gekennzeichnet, dass** das Röntgengerät eine C-Bogen-Röntgeneinrichtung ist.

8. Verfahren nach einem der vorhergehenden Ansprüche, **dadurch gekennzeichnet, dass** der dreidimensionale Bilddatensatz mittels eines Computertomographen, eines Magnetresonanztomographen oder eines C-Bogen-Röntgengeräts aufgenommen wird.

9. Verfahren nach einem der vorhergehenden Ansprüche, **dadurch gekennzeichnet, dass** der dreidimensionale Bilddatensatz vor dem erfindungsgemäßen Verfahren für die Zurverfügungstellung einer dreidimensionalen Gefäßstruktur und einer dreidimensionalen Knochenstruktur segmentiert wurde.

10. Verfahren nach einem der vorhergehenden Ansprüche, **dadurch gekennzeichnet, dass** die Überlagerung einer Vorwärtsprojektion (31; 32; 33) unter der optimalen Projektionsgeometrie (24) mit einem Live-Röntgenbild nicht weiter auf einem Bildschirm angezeigt wird, wenn eine Bewegung des Röntgengerätes, eine Änderung der Aufnahmegeometrie eines Live-Röntgenbildes oder/und eine Verlagerung eines Patienten auf einer Patientenliege festgestellt wurden.

11. Verfahren nach einem der vorhergehenden Ansprüche, **dadurch gekennzeichnet, dass** in einem Live-Röntgenbild wenigstens ein Messfeld festgelegt ist, dass das Messfeld eine sich möglicherweise zeitlich verändernde Struktur eng eingrenzt und dass für das Live-Röntgenbild eine Auswertung derart erfolgt, dass ein durch Addition aller Pixelwerte im Messfeld erhaltener Summenwert überwacht wird und bei einer vorgegebenen prozentualen Veränderung des Summenwertes gegenüber dem Summenwert eines vorherigen Live-Röntgenbildes eine Überlagerung einer Vorwärtsprojektion unter der optimalen Projektionsgeometrie (24) mit dem Live-Röntgenbild am Bildschirm nicht weiter erfolgt.

12. Verfahren nach einem der vorhergehenden Ansprüche, **dadurch gekennzeichnet, dass** die Gefäßstruktur mit oder ohne eine Kontur oder nur eine Kontur unter Verwendung der Vorwärtsprojektion (31; 32; 33) des Gefäßstrukturmodells 22 bei der optimalen Projektionsgeometrie (24) auf einem Anzeigegerät mit einem Live-Röntgenbild überlagert dargestellt werden.

**Claims**

1. A computer-implemented method of processing a three-dimensional image data set (11) of two-dimensional X-ray images from an X-ray apparatus, comprising the steps of:

   • Providing a three-dimensional image data set (11) of at least one examination area in which anatomical structures are present,
   • segmenting the three-dimensional image data set (11) to provide a three-dimensional vascular structure model (22) describing a vascular structure and a three-dimensional bone structure model (21) describing a bone structure,
   • acquiring, by means of the X-ray apparatus, a first two-dimensional X-ray image containing at least partially the bone structure and at least partially the vascular structure, under an acquisition geometry and the presence of a first contrast agent concentration,
   • Acquiring, by means of the X-ray apparatus, a second two-dimensional X-ray image of the examination area, which contains a second contrast medium concentration different from the first X-ray image, wherein the acquisition of the second X-ray image takes place under the same acquisition geometry as the acquisition of the first X-ray image,
   • subtracting the first and second X-ray images to produce and provide a subtraction image, the subtraction image including a vascular structure,
   • Determining an optimal projection geometry (24) using a threepart objective function (51),

     ▪ wherein a first part of the objective function (51) represents a similarity between a forward projection (31; 32; 33) of the bone structure model (21) of the three-dimensional image data set (11) and the bone structure in the first and/or second X-ray image under variation of an assumed projection geometry ($P_i$),
     ▪ wherein a second part of the objective function (51) represents a similarity between the forward projection (31; 32; 33) of the vessel structure model (22) of the three-dimensional image data set (11) and the vessel structure of the subtraction image under variation of the assumed projection geometry (24),
     ▪ and wherein a third part (23) of the objective function (51) represents a similarity between all image information of the forward projection (31; 32; 33) of the three-dimensional image data set (11) and all image information of the first and/or second X-ray image under variation of the assumed projection geometry (24); wherein

   • the optimal projection geometry (24) results from a minimisation or maximisation (53) of the objective function (51).

2. A method according to claim 1, **characterised in that** the target function (51) and/or parts of the target functions (51) are compared with a predeterminable threshold criterion.

3. A method according to claim 2, **characterised in that** if the threshold criterion of the objective function (51) is missed, the method is aborted and a user is offered switching to an alternative two-dimensional method.

4. A method according to any one of the preceding claims, **characterised in that** the three parts of the objective function (51) are weighted differently to determine the optimum projection geometry (24).

5. A method according to any one of the preceding claims, **characterised in that** a digital subtraction angiography is generated and used instead of a second two-dimensional X-ray image of the examination area.

6. A method according to claim 5, **characterised in that** the digital subtraction angiography is summarised by a minimum or maximum method.

7. A method according to any one of the preceding claims, **characterised in that** the X-ray apparatus is a C-arm X-ray apparatus.

8. A method according to any one of the preceding claims, **characterised in that** the three-dimensional image data set is acquired by means of a computer tomograph, a magnetic resonance tomograph or a C-arm X-ray machine.

9. A method according to any one of the preceding claims, **characterised in that** the three-dimensional image data set has been segmented prior to the method according to the invention for providing a three-dimensional vascular structure and a three-dimensional bone structure.

10. A method according to any one of the preceding claims, **characterised in that** the superposition of a forward projection (31; 32; 33) under the optimal projection geometry (24) with a live X-ray image is not further displayed on a screen when a movement of the X-ray apparatus, a change in the acquisition geometry of a live X-ray image or/and a displacement of a patient on a patient couch have been detected.

11. A method according to any one of the preceding claims, **characterised in that** at least one measuring field is defined in a live X-ray image, **in that** the measuring field narrowly delimits a possibly temporally changing structure, and **in that** an evaluation takes place for the live X-ray image in such a way that a sum value obtained by addition of all pixel values in the measuring field is monitored and, in the case of a predetermined percentage change in the sum value compared with the sum value of a previous live X-ray image, superimposition of a forward projection under the optimum projection geometry (24) with the live X-ray image on the screen does not take place any further.

12. A method according to any one of the preceding claims, **characterised in that** the vascular structure with or without a contour or only a contour using the forward projection (31; 32; 33) of the vascular structure model 22 at the optimal projection geometry (24) are displayed on a display device with a live X-ray image superimposed.

**Revendications**

1. Procédé mis en oeuvre par ordinateur pour le traitement d'un ensemble de données d'image tridimensionnelles (11) avec des radiographies bidimensionnelles d'un appareil à rayons X, comprenant les étapes suivantes :

   • mise à disposition d'un jeu de données d'image tridimensionnel (11) d'au moins une zone d'examen dans laquelle des structures anatomiques sont présentes,
   • segmenter le jeu de données d'image tridimensionnel (11) pour une mise à disposition d'un modèle de structure vasculaire tridimensionnel (22) décrivant une structure vasculaire et d'un modèle de structure osseuse tridimensionnel (21) décrivant une structure osseuse,
   • l'acquisition, au moyen de l'appareil radiographique, d'une première image radiographique bidimensionnelle contenant au moins partiellement la structure osseuse et au moins partiellement la structure vasculaire, sous une géométrie d'acquisition et la présence d'une première concentration d'agent de contraste,
• l'acquisition, au moyen de l'appareil radiographique, d'une deuxième image radiographique bidimensionnelle de la zone d'examen, qui contient une deuxième concentration en agent de contraste différente de la première image radiographique, l'acquisition de la deuxième image radiographique s'effectuant sous la même géométrie d'enregistrement que l'enregistrement de la première image radiographique,
• la soustraction de la première et de la deuxième image radiographique pour produire et fournir une image de soustraction, l'image de soustraction contenant une structure vasculaire,
• déterminer une géométrie de projection optimale (24) en utilisant une fonction cible en trois parties (51),

   ▪ dans lequel une première partie de la fonction cible (51) représente une similitude entre une projection vers l'avant (31 ; 32 ; 33) du modèle de structure osseuse (21) de l'ensemble de données d'image tridimensionnelle (11) et la structure osseuse dans la première et/ou la deuxième image radiographique en faisant varier une géométrie de projection supposée ($P_i$),
   ▪ dans lequel une deuxième partie de la fonction cible (51) représente une similitude entre la projection vers l'avant (31 ; 32 ; 33) du modèle de structure vasculaire (22) de l'ensemble de données d'image tridimensionnelle (11) et la structure vasculaire de l'image de soustraction, avec variation de la géométrie de projection supposée (24),
   ▪ et dans lequel une troisième partie (23) de la fonction cible (51) représente une similitude entre toutes les informations d'image de la projection vers l'avant (31 ; 32 ; 33) de l'ensemble de données d'image tridimensionnelle (11) et toutes les informations d'image de la première et/ou de la deuxième image radiographique en faisant varier la géométrie de projection supposée (24) ; dans lequel

   • la géométrie de projection optimale (24) résulte d'une minimisation ou d'une maximisation (53) de la fonction cible (51).

2. Procédé selon la revendication 1, **caractérisé en ce que** la fonction cible (51) et/ou des parties des fonctions cibles (51) sont comparées à un critère de valeur seuil pouvant être prédéfini.

3. Procédé selon la revendication 2, **caractérisé en ce**

**que**, si le critère de seuil de la fonction cible (51) n'est pas atteint, le procédé est interrompu et il est proposé à un utilisateur de passer à un procédé bidimensionnel alternatif.

4. Procédé selon l'une des revendications précédentes, **caractérisé en ce que**, pour déterminer la géométrie de projection optimale (24), les trois parties de la fonction cible (51) sont pondérées différemment.

5. Procédé selon l'une des revendications précédentes, **caractérisé en ce qu'**au lieu d'une deuxième radiographie bidimensionnelle de la zone d'examen, on produit et on utilise une angiographie numérique par soustraction.

6. Procédé selon la revendication 5, **caractérisé en ce que** l'angiographie par soustraction numérique est résumée par une procédure de minimum ou de maximum.

7. Procédé selon l'une des revendications précédentes, **caractérisé en ce que** l'appareil de radiographie est un appareil de radiographie à arc en C.

8. Procédé selon l'une des revendications précédentes, **caractérisé en ce que** l'ensemble de données d'images tridimensionnelles est acquis au moyen d'un tomographe assisté par ordinateur, d'un tomographe à résonance magnétique ou d'un appareil radiographique à arc en C.

9. Procédé selon l'une quelconque des revendications précédentes, **caractérisé en ce que** le jeu de données d'image tridimensionnelle a été segmenté avant le procédé selon l'invention pour la mise à disposition d'une structure vasculaire tridimensionnelle et d'une structure osseuse tridimensionnelle.

10. Procédé selon l'une des revendications précédentes, **caractérisé en ce que** la superposition d'une projection vers l'avant (31 ; 32 ; 33) sous la géométrie de projection optimale (24) avec une image radiographique en direct n'est plus affichée sur un écran lorsqu'un mouvement de l'appareil radiographique, une modification de la géométrie de prise de vue d'une image radiographique en direct ou/et un déplacement d'un patient sur un lit de patient ont été constatés.

11. Procédé selon l'une des revendications précédentes, **caractérisé en ce qu'**au moins un champ de mesure est défini dans une image radiographique en direct, **en ce que** le champ de mesure délimite étroitement une structure qui se modifie éventuellement dans le temps et **en ce que**, pour l'image radiographique en direct, une évaluation a lieu de telle sorte qu'une valeur de somme obtenue par addition de toutes les valeurs de pixels dans le champ de mesure est surveillée et, en cas de modification prédéfinie en pourcentage de la valeur de somme par rapport à la valeur de somme d'une image radiographique en direct précédente, une superposition d'une projection vers l'avant sous la géométrie de projection optimale (24) avec l'image radiographique en direct sur l'écran n'a plus lieu.

12. Procédé selon l'une des revendications précédentes, **caractérisé en ce que** la structure vasculaire est représentée avec ou sans un contour ou seulement un contour en utilisant la projection vers l'avant (31 ; 32 ; 33) du modèle de structure vasculaire 22 pour la géométrie de projection optimale (24) sur un appareil d'affichage avec une image radiographique en direct superposée.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016203857 A1
- US 20200051258 A1
- US 20060188139 A
- US 2011052026 A1
- US 20180042566 A1